# EUROPEAN PATENT SPECIFICATION

(11) **EP 1 359 928 B1**
(45) Date of publication and mention of the grant of the patent: **14.12.2005**
(21) Application number: 02710559.2
(22) Date of filing: 24.01.2002
(51) Int. Cl.: A61K 35/78, A61K 7/48, A61K 31/12

(54) **MEDICINAL AND COSMETIC USE OF HOP AND CO-ENZYME Q10**
MEDIZINISCHE UND KOSMETISCHE VERWENDUNG VON HOPFEN UND COENZYM Q10
UTILISATION DU HOUBLON ET DU COENZYME Q10 A DES FINS MEDICALES ET COSMETIQUES

(30) Priority: 26.01.2001 NL 1017205
(43) Date of publication of application: 12.11.2003
(73) Proprietor: B.A.S.A. SARL, 9206 Diekirch (LU)
(72) Inventor: Van de Wiel, Adriaan Emanuel Hendricus Anna Maria, 6718 TB Ede (NL); Christiaans, Marijke, 6718 TB Ede (NL)
(74) Representative: Jilderda, Anne Ayolt
(86) International application number: PCT/NL2002/000054
(87) International publication number: WO 2002/078727

(56) References cited:
- LOCKWOOD K ET AL: "Apparent partial remission of breast cancer in 'high risk' patients supplemented with nutritional antioxidants, essential fatty acids and coenzyme Q10" MOLECULAR ASPECTS OF MEDICINE, PERGAMON PRESS, OXFORD, GB, vol. 15, no. SUPPL, 1994, pages S231-S240, XP002108766 ISSN: 0098-2997
- DATABASE BIOSIS [Online] BIOSCIENCES INFORMATION SERVICE, PHILADELPHIA, PA, US; June 2000 (2000-06) PORTAKAL OYTUN ET AL: "Coenzyme Q10 concentrations and antioxidant status in tissues of breast cancer patients." Database accession no. PREV200000488189 XP002180330 & CLINICAL BIOCHEMISTRY, vol. 33, no. 4, June 2000 (2000-06), pages 279-284, ISSN: 0009-9120
- DATABASE WPI Section Ch, Week 197803 Derwent Publications Ltd., London, GB; Class B04, AN 1978-05666A XP002180331 & JP 52 145509 A (MATSUI T), 3 December 1977 (1977-12-03)
- DATABASE WPI Section Ch, Week 199006 Derwent Publications Ltd., London, GB; Class B05, AN 1990-037332 XP002180332 & CN 1 031 021 A (TIANJIN INST COSMET), 15 February 1989 (1989-02-15)

## Description

The present invention relates to a composition which contains hop Q10 and a zinc salt such as zinc nitrate or zinc oxalate and which can be used as medication or cosmetic agent, in particular to suppress and prevent malignant cancer cells and benign cysts.

Such an agent with anti-cancer action is known from J. Agric. Food Chem., 47 (1), 1999 and from the international patent application WO 00/53205. There does of course continue to exist a need for other agents based on natural substances with a better action against cancer.

The present invention provides a composition of the type stated in the preamble which also contains co-enzyme Q10 in addition to hop. It is known that co-enzyme Q10 greatly improves the vitality and physical stamina. Co-enzyme Q10 has unexpectedly now been found to also have a synergistic effect on the activity of hop against cancerous growths and in causing the subsidence of benign cysts. The composition containing hop co-enzyme Q10 and a zinc salt also works in one or more of the following cases: in preventing and aiding recovery from lumpy breast tissue, reducing the lumpy appearance of and strengthening breast tissue. The weight ratio of hop to co-enzyme Q10 in the composition preferably amounts to 0.75 to 75, more preferably to between 2.5 and 10.

A particular embodiment also contains one or more of the following substances with a favourable influence on the anti-carcinogenic or cosmetic action of the composition or on the state of health of the user generally, i.e. buckwheat and rye. The weight ratio between hop and buckwheat preferably amounts to 1.5 to 35, more preferably to between 2.5 and 10. The weight ratio between hop and zinc salt preferably further amounts to 0.75 and 10, more preferably to about 2.5 and 7.5. The weight ratio between hop and rye preferably amounts to between 1 and 15, more preferably to between 2 and 5.

It is assumed that the constituents of hop, genistein and daidzein, which are among the phyto-oestrogenic substances or isoflavonoids, have tumor-inhibiting action. Just as the oestrogens occurring naturally in the body, genistein can bond to the oestrogen receptor of the damaged cell, whereby further division is inhibited. Tumors which depend for their development on oestrogen, such as that in breast tissue, the prostate and the intestines, are thus inhibited in their growth. This theory is supported by a low mortality rate from breast cancer among women in Asia, this being generally attributed to the phyto-oestrogenic activity of isoflavonoids such as genistein and daidzein from soya, much more of which is consumed than in the western world.

Co-enzyme Q10 is known to function as electron transporter in the respiratory cycle of the mitochondria of the cell, wherein it optimizes the so-called A.T.P. production. The A.T.P. (adenosine triphosphate) is the storage site of cellular energy. Ingestion of co-enzyme Q10 improves the activity of every body cell, which particularly manifests itself in the organs which are greatly dependent on energy provision such as heart and liver, and this is expressed in an improvement of diverse heart conditions, diabetes, overweight, paradontosis and paradontitis, gastric ulcers, muscular dystrophy and fatigue. The pharmaceutical effect of this co-enzyme per se or in synergy with hop for the purpose of preventing and combatting malignant as well as benign tumors is however nowhere described or even suggested.

The addition of buckwheat and/or rye is further recommended because of the favourable influence thereof on the bowel function, wherein buckwheat forms a source of vitamin B and rye a source of vitamin D. Finally, a zinc salt, for instance such as zinc citrate or zinc oxolate is also included in the composition according to the invention, since zinc ions reduce stress, fatigue and susceptibility to infections.

It is self-evident that the hop is preferably used in the most natural form, for instance as unprocessed hop flowers. The action of the above mentioned agricultural products will be shown to best effect when they are used in the form in which they are harvested, i.e. as grain including the skins. These fibres restore the flora of the intestines and therefore contribute to the body's own immune system.

A medication in different forms can be prepared from the composition according to the invention, both in dry form and in liquid form. However, a particularly practical embodiment of the medication has the feature that it is embodied in capsule or tablet form, wherein one or more excipients are normally used, such as for instance silicon oxide, calcium phosphate, magnesium stearate, oats or starch. This medication can optionally also be ingested in solution or as suspension.

Good results have been achieved with an application of the medication according to the invention which is characterized in that the daily dosage thereof amounts at least to 250-1250 mg hop, 60-300 mg co-enzyme Q10 and 50-250 mg zinc citrate or an equivalent amount of another zinc salt, such as for instance zinc oxolate; preferably supplemented with one or more of the following compounds: 60 - 300 mg buckwheat, and 80 - 400 mg rye.

A particular embodiment according to the invention is formed by a tablet or capsule which contains per 500 mg at least 125 mg hop, 30 mg co-enzyme Q10, 30 mg buckwheat, 25 mg zinc citrate or an equivalent amount of another zinc salt, 40 mg rye and optional excipients. A tablet or capsule preferably contains per 500 mg of active substances 250 mg hop, 60 mg co-enzyme Q 10, 60 mg buckwheat, 50 mg zinc citrate or an equivalent amount of another zinc salt, and 80 mg rye.

The preferable point of departure in respect of the composition is purely natural products and components obtained from natural products. This does however have the drawback that the concentrations of active substances are sometimes low and that a large amount of the medication therefore has to be ingested to ensure sufficient effect. A particular embodiment of the medication has in this respect the feature that the agent in dry form is processed into capsules or tablets with a minimum of 80% by weight of active substance and for the rest an excipient, wherein the capsules or tablets preferably have a weight which does not exceed 850 milligrams. It has been established that a capsule or tablet under 850 milligrams can still be easily ingested, while a heavier capsule or a heavier tablet could on occasion result in problems with swallowing. A maximum quantity of the agent is thus still administered per capsule or tablet in acceptable manner. Also falling within the scope of the invention is the preparation of a solution or suspension acting in one or more of the following cases: for the purpose of suppressing or preventing malignant cancer cells or benign cysts and the growth thereof, for preventing lumpy breast tissue of for strengthening breast tissue, wherein a tablet or capsule as described above is mixed with water to form such a solution or suspension.

The invention will now be further elucidated with reference to an embodiment:

### Example

Per 1000 grams of active substance to be prepared, about 585 grams hop, 140 grams buckwheat, 187 grams rye, and 117 grams zinc citrate are mixed in a container and ground to an homogeneous mixture. About 140 grams of co-enzyme Q10 is added hereto per 1000 grams of active substance to be prepared. This composition is mixed in a ratio of 70:12 with an excipient consisting of silicon oxide, calcium phosphate and magnesium stearate, and processed to capsules of 500 mg each. Preferably natural flavourings can optionally be added to conceal the strong hop taste in these capsules.

1 tot 5 capsules per day are ingested for 5 to 9 months, which corresponds to a daily dosage of 250 - 1250 mg of hop and 60 - 300 mg of co-enzyme Q10. At least a stabilization and in a number of cases also a decrease in the size of the tumors of cysts can be observed after a period of one to several months.

### Comparative example

A capsule is prepared as in the example above, but an excipient is then used instead of co-enzyme Q10. The activity is tested in the same way. The stabilization and decrease in size of the tumors or cysts is considerably less than that found after use of the composition described in the example.

## Claims

1. Composition for use as medication or as a cosmetic agent comprising hop, Q10 and a zinc salt.

2. Composition according to claim 1, wherein the weight ratio of hop to co-enzyme Q10 amounts to .75 to 75.

3. Composition according to claim 1 or 2, comprising buckwheat.

4. Composition according to claim 3, wherein the weight ratio of hop to buckwheat amounts to 1.5 to 35.

5. Composition according to claim 1, wherein zinc citrate or zinc oxolate is present as zinc salt.

6. Composition according to claim 5, wherein the weight ratio of hop to zinc citrate amounts to .75 to 10.

7. Composition according to any of the preceding claims, comprising rye.

8. Composition according to claim 7, wherein the weight ratio of hop to rye amounts to I to 15.

9. Use of a composition according to any of the preceding claims for the preparation of an agent for the purpose of preventing or aiding recovery from lumpy breast tissue or for strengthening the breast tissue.

10. Use of a composition according to any of the claims 1 - 8 for the preparation of a medication for suppressing and preventing malignant cancer cells or benign cysts and the growth thereof.

11. Use according to claim 10 for suppressing and preventing malignant cancer cells or benign cysts in the female breast or in the prostate.

12. Use according to any of the claims 9-11, wherein the daily dosage of the medication amounts to 250 to 1250 mg hop and 60 to 300 mg co-enzyme Q10.

13. Use according to claim 12, wherein the daily dosage also amounts to 60 to 300 mg buckwheat.

14. Use according to claim 12 or 13, wherein the daily dosage also amounts to 50 to 250 mg zinc citrate or an equivalent amount of another zinc salt.

15. Use according to any of the claims 12 - 14, wherein the daily dosage also amounts to 80 to 400 mg rye.

16. Use according to claim 15, wherein the daily dosage amounts to at least 250 mg hop, 60 mg co-enzyme Q10, 60 mg buckwheat, 50 mg zinc citrate or an equivalent amount of another zinc salt, and 80 mg rye.

17. Tablet or capsule containing hop, co-enzyme Q10, buckwheat, a zinc salt, rye and optional excipients.

18. Tablet or capsule according to claim 17, containing per 500 mg at least 125 mg hop, 30 mg co-enzyme Q10, 30 mg buckwheat, 25 mg zinc citrate or an equivalent amount of another zinc salt, 40 mg rye and optional excipients.

19. Tablet or capsule according to claim 18, containing per 500 mg of active substances 250 mg hop, 60 mg co-enzyme Q10, 60 mg buckwheat, 50 mg zinc citrate or an equivalent amount of another zinc salt and 80 mg rye.

20. Method of preparing a solution or suspension for the purpose of suppressing and preventing malignant cancer cells or benign cysts and the growth thereof or for preventing or aiding recovery from lumpy breast tissue or for strengthening breast tissue, wherein a tablet or capsule as described in any of the claims 17 - 19 is mixed with water.

## Patentansprüche

1. Zusammensetzung zur Verwendung als Medikament oder als kosmetisches Agens, die Hopfen, Q10 und ein Zinksalz aufweist.

2. Zusammensetzung gemäß Anspruch 1, wobei das Gewichtsverhältnis von Hopfen zu Co-Enzym Q10 0,75 zu 75 beträgt.

3. Zusammensetzung gemäß Anspruch 1 oder 2, die Buchweizen aufweist.

4. Zusammensetzung gemäß Anspruch 3, wobei das Gewichtsverhältnis von Hopfen zu Buchweizen 1,5 zu 35 beträgt.

5. Zusammensetzung nach Anspruch 1, wobei als Zinksalz Zinkcitrat oder Zink-Oxolat vorhanden ist.

6. Zusammensetzung gemäß Anspruch 5, wobei das Gewichtsverhältnis von Hopfen zu Zinkcitrat 0,75 zu 10 beträgt.

7. Zusammensetzung gemäß einem der vorhergehenden Ansprüche, die Roggen aufweist.

8. Zusammensetzung gemäß Anspruch 7, wobei das Gewichtsverhältnis von Hopfen zu Roggen 1 zu 15 beträgt.

9. Verwendung einer Zusammensetzung gemäß einem der vorhergehenden Ansprüche zur Herstellung eines Agens zum Zwecke des Vorbeugens oder der Unterstützung der Genesung von knotigem Brustgewebe oder zur Stärkung des Brustgewebes.

10. Verwendung einer Zusammensetzung gemäß einem der Ansprüche 1 bis 8 zur Herstellung eines Medikamentes zur Unterdrückung und Vorbeugung von bösartigen Krebszellen oder gutartigen Zysten und deren Wachstum.

11. Verwendung gemäß Anspruch 10 zur Unterdrückung und Vorbeugung bösartiger Krebszellen oder gutartiger Zysten in der weiblichen Brust oder in der Prostata.

12. Verwendung gemäß einem der Ansprüche 9 bis 11, wobei die tägliche Dosis des Medikamentes 250 bis 1250 mg Hopfen und 60 bis 300 mg Co-Enzym Q10 beträgt.

13. Verwendung gemäß Anspruch 12, wobei die tägliche Dosis ebenfalls 60 bis 300 mg Buchweizen beträgt.

14. Verwendung gemäß Anspruch 12 oder 13, wobei die tägliche Dosis ebenfalls 50 bis 250 mg Zinkcitrat oder eine äquivalente Menge eines anderen Zinksalzes beträgt.

15. Verwendung gemäß einem der Ansprüche 12 bis 14, wobei die tägliche Dosis ebenfalls 80 bis 400 mg Roggen beträgt.

16. Verwendung gemäß Anspruch 15, wobei die tägliche Dosis zumindest 250 mg Hopfen, 60 mg Co-Enzym Q10, 60 mg Buchweizen, 50 mg Zinkcitrat oder eine äquivalente Menge eines anderen Zinksalzes, und 80 mg Roggen beträgt.

17. Tablette oder Kapsel enthaltend Hopfen, Co-Enzym Q10, Buchweizen, ein Zinksalz, Roggen und optional Trägermittel.

18. Tablette oder Kapsel gemäß Anspruch 17, die pro 500 mg zumindest 125 mg Hopfen, 30 mg Co-Enzym Q10, 30 mg Buchweizen, 25 mg Zinkcitrat oder eine äquivalente Menge eines anderen Zinksalzes, 40 mg Roggen und optional Trägermittel enthält.

19. Tablette oder Kapsel gemäß Anspruch 18, die pro 500 mg von aktiven Substanzen 250 mg Hopfen, 60 mg Co-Enzym Q10, 60 mg Buchweizen, 50 mg Zinkcitrat oder eine äquivalente Menge eines anderen Zinksalzes, und 80 mg Roggen enthält.

20. Verfahren zur Herstellung einer Lösung oder Suspension zum Zwecke des Unterdrückens und Vorbeugens von bösartigen Krebszellen oder gutartigen Zysten und deren Wachstum, oder zur Vorbeugung oder Unterstützung der Genesung von knotigem Brustgewebe oder zur Stärkung von Brustgewebe, wobei eine in einem der Ansprüche 17 bis 19 beschriebene Tablette oder Kapsel mit Wasser gemischt wird.

## Revendications

1. Composition pour une utilisation comme médicament ou comme agent cosmétique comprenant du houblon, du Q10, et un sel de zinc.

2. Composition selon la revendication 1, dans laquelle le rapport en poids du houblon sur le co-enzyme Q10 est compris entre 0,75 et 75.

3. Composition selon la revendication 1 ou 2, comprenant du sarrasin.

4. Composition selon la revendication 3, dans laquelle le rapport en poids du houblon sur le sarrasin est compris entre 1,5 et 35.

5. Composition selon la revendication 1, dans laquelle du citrate de zinc ou de l'oxalate de zinc est présent en tant que sel de zinc.

6. Composition selon la revendication 5, dans laquelle le rapport en poids du houblon sur le citrate de zinc est compris entre 0,75 et 10.

7. Composition selon l'une quelconque des revendications précédentes, comprenant du seigle.

8. Composition selon la revendication 7, dans laquelle le rapport en poids du houblon sur le seigle est compris entre 1 et 15.

9. Utilisation d'une composition selon l'une quelconque des revendications précédentes pour la préparation d'un agent dans le but de prévenir ou d'aider à la récupération d'un tissu du sein granuleux ou pour renforcer le tissu du sein.

10. Utilisation d'une composition selon l'une quelconque des revendications 1-8 pour la préparation d'un médicament pour supprimer et prévenir des cellules cancéreuses malignes ou des kystes bénins et leur croissance.

11. Utilisation selon la revendication 10 pour supprimer et prévenir des cellules cancéreuses malignes ou des kystes bénins dans le sein femelle ou dans la prostate.

12. Utilisation selon l'une quelconque des revendications 9-11, dans laquelle la posologie quotidienne de médicament est comprise entre 250 et 1250 mg de houblon et entre 60 et 300 mg de co-enzyme Q10.

13. Utilisation selon la revendication 12, dans laquelle la posologie quotidienne comprend aussi 60 à 300 mg de sarrasin.

14. Utilisation selon la revendication 12 ou 13, dans laquelle la posologie quotidienne comprend aussi 50 à 250 mg de citrate de zinc ou une quantité équivalente d'un autre sel de zinc.

15. Utilisation selon l'une quelconque des revendications 12-14, dans laquelle la posologie quotidienne comprend aussi 80 à 400 mg de seigle.

16. Utilisation selon la revendication 15, dans laquelle la posologie quotidienne s'élève à au moins 250 mg de houblon, 60 mg de co-enzyme Q10, 60 mg de sarrasin, 50 mg de citrate de zinc ou d'une quantité équivalente d'un autre sel de zinc et 80 mg de seigle.

17. Comprimé ou capsule contenant du houblon, du co-enzyme Q10, du sarrasin, un sel de zinc, du seigle et des excipients éventuels.

18. Comprimé ou capsule selon la revendication 17, contenant pour 500 mg au moins 125 mg de houblon, 30 mg de co-enzyme Q10, 30 mg de sarrasin, 25 mg de citrate de zinc ou d'une quantité équivalente d'un autre sel de zinc et 40 mg de seigle et des excipients éventuels.

19. Comprimé ou capsule selon la revendication 18, contenant pour 500 mg de substances actives 250 mg de houblon, 60 mg de co-enzyme Q10, 60 mg de sarrasin, 50 mg de citrate de zinc ou d'une quantité équivalente d'un autre sel de zinc et 80 mg de seigle.

20. Procédé de préparation d'une solution ou d'une suspension dans le but de supprimer et de prévenir des cellules cancéreuses malignes ou des kystes bénins et leur croissance ou pour prévenir ou aider à la récupération d'un tissu du sein granuleux ou pour renforcer le tissu du sein, dans lequel un comprimé ou une capsule tels que décrits dans l'une quelconque des revendications 17-19 est mélangé avec de l'eau.
